# EUROPEAN PATENT APPLICATION

(11) **EP 1 211 274 A1**
(43) Date of publication of application: **05.06.2002**
(21) Application number: 01128072.4
(22) Date of filing: 26.11.2001
(51) Int. Cl.: C08G 63/08, C07D 315/00

(54) **Method for producing aliphatic polyester**

(30) Priority: 01.12.2000 JP 2000367302; 05.12.2000 JP 2000370258; 05.12.2000 JP 2000370259; 12.04.2001 JP 2001114044; 20.04.2001 JP 2001122694; 20.04.2001 JP 2001122695; 16.07.2001 JP 2001215391
(71) Applicant: CANON KABUSHIKI KAISHA, Ohta-ku, Tokyo (JP)
(72) Inventor: Minami, Masato, Canon Kabushiki Kaisha, Tokyo (JP); Kozaki, Shinya, Canon Kabushiki Kaisha, Tokyo (JP)
(74) Representative: Weser, Wolfgang, Dr. Dipl.-Phys.

(57) **Abstract**

The invention relates to a method for producing an aliphatic polyester, utilizing starch as a raw material.

The invention produces an aliphatic polyester by the steps of hydrolyzing starch to obtain glucose, oxidizing the glucose to obtain gluconolactone or gluconic acid, reducing the gluconolactone or the gluconic acid to obtain caproic acid, chlorinating the caproic acid to obtain 6-chlorocaproic acid, cyclizing the 6-chlorocaproic acid to obtain ε-caprolactone, and executing ring-opening polymerization of the ε-caprolactone.

## Description

### BACKGROUND OF THE INVENTION

### Field of the Invention

The present invention relates to a method for producing aliphatic polyester and a method for using starch as a resource.

### Related Background Art

The conventional general-purpose plastic products are composed of polymers synthesized from petroleum resources. More specifically, polymer products such as polyester, polystyrene, nylon, polyethylene, polyvinyl chloride, polyimide, polycarbonate etc. are all synthesized from petroleum. However, the petroleum is a limited resource which is to run out sooner or later. For this reason there is strongly desired a technology for producing the general-purpose plastic products from a new raw material capable of substituting petroleum, namely a recyclable raw material.

On the other hand, starch is a polymer compound formed by dehydration polymerization of D-glucose, and is an important polysaccharide comparable to cellulose. Starch is produced from potato, sweet potato, corn etc., with the worldwide production (production amount of corn) amounting to 400 to 500 million tons per year, and is a recyclable resource having the largest production amount among the natural resources. Starch can highly be expected as a new resource which replaces the petroleum, if general-purpose plastic products can be produced therefrom.

### SUMMARY OF THE INVENTION

The object of the present invention is to provide a method for producing aliphatic polyester utilizing starch as a raw material.

Based on a standpoint that a novel technical development is required to provide against the exhaustion of the petroleum resources in the future, the present inventors through intensive investigation have noticed starch as a raw material which can replace petroleum and have found that aliphatic polyester can be synthesized from caproic acid that can be obtained from starch via glucose, thereby attaining the present invention. This finding opens up a way for utilizing starch as an efficient resource in obtaining plastics of high quality from starch as a starting material.

The above-mentioned object can be attained, according to an embodiment of the present invention, by a method for producing an aliphatic polyester represented by the following formula (I): 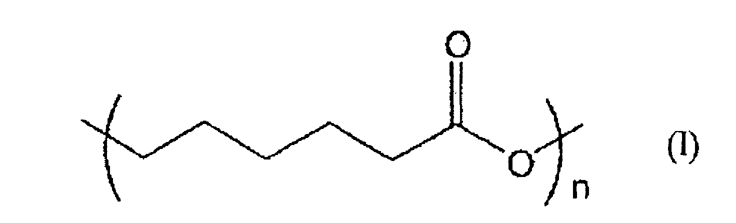 (wherein n stands for an integer within a range from 5 to 10,000), the method comprising the steps of:
(i) hydrolyzing starch to obtain glucose;
(ii) oxidizing the glucose to obtain gluconolactone;
(iii) reducing the gluconolactone to obtain caproic acid;
(iv) chlorinating the caproic acid to obtain 6-chlorocaproic acid;
(v) cyclizing the 6-chlorocaproic acid to obtain ε-caprolactone represented by the following formula (II): 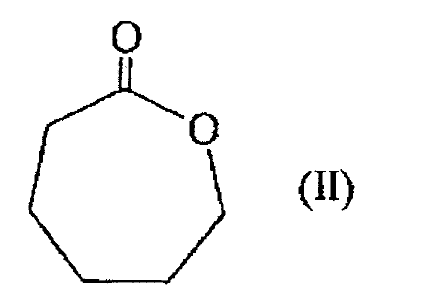 and
(vi) executing ring-opening polymerization of the ε-caprolactone.

The aforementioned object can be attained also, in another embodiment of the present invention, by a method for producing an aliphatic polyester represented by the following formula (I): 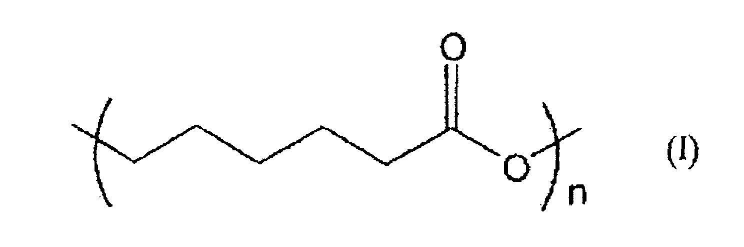 (wherein n stands for an integer within a range from 5 to 10,000), the method comprising the steps of:
(i) hydrolyzing starch to obtain glucose;
(ii) oxidizing the glucose to obtain gluconic acid;
(iii) reducing the gluconic acid to obtain caproic acid;
(iv) chlorinating the caproic acid to obtain 6-chlorocaproic acid;
(v) cyclizing the 6-chlorocaproic acid to obtain ε-caprolactone represented by the following formula (II): 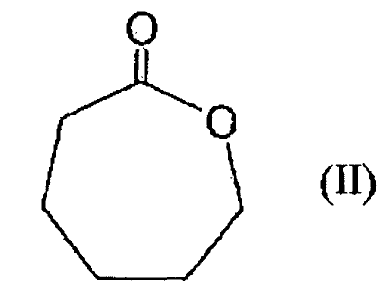 and
(vi) executing ring-opening polymerization of the ε-caprolactone.

ε-caprolactone is a compound having an intramolecular cyclic ester structure and is well known as an industrially producible compound by oxidizing cyclohexanone. It is also known that ε-caprolactone easily undergoes ring-opening polymerization to provide aliphatic polyester' (Japanese Patent Application Laid-Open No. 11-158172). However, there have not been known examples, except that of the present inventors, of synthesizing ε-caprolactone from starch and obtaining aliphatic polyester therefrom.

### DETAILED DESCRIPTION OF THE PREFERRED EMBODIMENTS

The method of the present invention for producing aliphatic polyester comprises the steps of:
(i) hydrolyzing starch to obtain glucose;
(ii) oxidizing the glucose to obtain gluconolactone or gluconic acid;
(iii) reducing the gluconolactone or the gluconic acid to obtain caproic acid;
(iv) chlorinating the caproic acid to obtain 6-chlorocaproic acid;
(v) cyclizing the 6-chlorocaproic acid to obtain ε-caprolactone; and
(vi) executing ring-opening polymerization of the ε-caprolactone to obtain aliphatic polyester represented by the foregoing formula (I).

The method for synthesizing aliphatic polyester from starch opens up a novel way of utilizing starch as a resource. From this standpoint, the method of the present invention for producing aliphatic polyester also provides a useful method for utilizing starch as a new resource.

In the following there will be explained each of the aforementioned steps (i) to (vi).

### Step (i) (starch to glucose)

Conversion from starch to glucose can be achieved for example by hydrolysis with a dilute acid such as sulfuric acid, hydrolysis with an enzyme such as amylase or maltase, or hydrolysis with ultracritical water. The step of obtaining glucose from starch is preferably executed by hydrolysis with an acid. The reaction conditions can be suitably determined according to the already known method.

### Step (ii) (glucose to gluconolactone or gluconic acid)

Conversion from glucose to gluconolactone can be achieved for example by bromine oxidation of glucose or by a method utilizing notatin which is a glucose oxidase. The step of obtaining gluconolactone from glucose is preferably executed by bromine oxidation. The reaction conditions can be suitably determined according to the already known method.

Conversion from glucose to gluconic acid can be achieved for example by oxidation with bromine and concentrated sulfuric acid, more specifically oxidizing and hydrolyzing glucose in sulfuric acid saturated with bromine, or by electrolytic oxidation of a glucose solution or by fermentation of gluconic acid utilizing bacteria of Penicillium family. The step of obtaining gluconic acid from glucose is preferably executed by oxidation utilizing bromine and concentrated sulfuric acid. The reaction conditions can be suitably determined according to the already known method.

### Step (iii) (gluconolactone or gluconic acid to caproic acid)

Conversion of gluconolactone or gluconic acid to caproic acid can be achieved for example by reduction thereof with hydroiodic acid and red phosphorus. In this reaction, it is desirable that the hydroxyl radical alone of gluconolactone or gluconic acid is oxidized.

The amount of red phosphorus employed in the reduction is preferably 1.8 to 2.4 equivalents with respect to gluconolactone or gluconic acid. Hydroiodic acid employed in the reduction preferably has a concentration of 50 to 60 mass%, and is preferably employed in a weight of 40 to 60 times with respect to the weight of gluconolactone or gluconic acid. The reducing reaction is completed by refluxing gluconolactone or gluconic acid and red phosphorus in hydroiodic acid for about 20 hours. The reaction mixture is filtered, then the filtrate is extracted with ether and washed with an aqueous solution of sodium hydrosulfite of about 5 mass%, and caproic acid can be obtained by distilling the ether solvent and executing vacuum distillation.

### Step (iv) (caproic acid to 6-chlorocaproic acid)

Conversion from caproic acid to 6-chlorocaproic acid can be achieved for example by chlorination with chlorine and concentrated sulfuric acid, preferably by chlorination conducted by reacting caproic acid with chlorine in concentrated sulfuric acid. The reaction conditions can be suitably determined according to the known method.

### Step (v) (6-chlorocaproic acid to ε-caprolactone)

Conversion from 6-chlorocaproic acid to ε-caprolactone can be achieved for example by cyclization utilizing an aqueous solution of sodium hydroxide, preferably by boiling 6-chlorocaproic acid in an aqueous solution of sodium hydroxide. The reaction conditions can be suitably determined according to the known method.

### Step (vi) (ε-caprolactone to aliphatic polyester; ring-opening polymerization)

In the present invention, aliphatic polyester is synthesized by ring-opening polymerization of ε-caprolactone utilizing a compound having a hydroxyl radical as an initiator normally in the presence of a catalyst. The initiator is used for opening the ring of ε-caprolactone, and the catalyst accelerates the polymerization by interacting with the ring-opened product.

### (Polymerization catalyst)

In the present invention, a known ring-opening polymerization catalyst can be employed as the polymerization catalyst in the ring-opening polymerization of ε-caprolactone. Examples of such catalyst include tin dichloride, tin tetrachloride, tetra-n-butoxy-germanium, tetramethoxy-germanium, tetraethoxy-germanium, triethoxy-aluminum, tri-n-propoxy-aluminum, tri-iso-propoxy-aluminum, tri-n-butoxy-aluminum, triiso-butoxy-aluminum, aluminum chloride, triethyl-aluminum, trimethyl-aluminum, di-iso-propyl zinc, dimethyl-zinc, diethyl-zinc, zinc chloride, tetra-n-propoxy-titanium, tetra-n-butoxy-titanium, tetra-t-butoxy-titanium, tetraethoxy-zirconium, tetramethoxy-zirconium, tetra-iso-propoxy-zirconium, tetra-n-butoxy-zirconium, tetra-iso-butoxy-zirconium and tetra-t-butoxy-zirconium. Such catalyst may be employed singly or as a mixture of at least two catalysts.

The amount of polymerization catalyst can be determined suitably, but is usually within a range of 0.01 to 10 wt.%, preferably 0.05 to 5 wt.% with respect to the total amount of ε-caprolactone and the polymerization initiator.

### (Polymerization initiator)

In the present invention, a known polymerization initiator can be employed in the ring-opening polymerization of ε-caprolactone. Examples of such polymerization initiator include monools such as methanol, ethanol, 1-propanol, 2-propanol, butanols or phenol, diols such as ethylene glycol, 1,3-propanediol, 1,4-butanediol, diethylene glycol, 1,5-pentanediol, 1,6-hexanediol, 1,7-heptanediol, 1,8-octanediol or 1,10-decanediol, triols such as glycerin or trimethylol propane, and polyols such as neopentyl glycol or pentaerythritol. Such initiator may be employed singly or as a mixture of at least two initiators.

The moler ratio of the polymerization initiator to be employed in the present invention and ε-caprolactone can be suitably selected according to the polymerization ratio of the desired aliphatic polyester, and is normally within a range of 1:1 to 1:5,000, preferably within a range of 1:1 to 1:2,000.

The ring-opening polymerization of ε-caprolactone can be executed by a polymerization reaction of ε-caprolactone in the presence of the polymerization catalyst and the polymerization initiator under the presence of inert gas or under a reduced pressure. The ring-opening polymerization of ε-caprolactone is preferably executed in a nitrogen atmosphere for the ease of operation.

In the ring-opening polymerization of ε-caprolactone, the reaction temperature and time can be arbitrarily selected. The reaction temperature is preferably equal to 50° C or higher, particularly 100° C or higher in order to obtain a sufficiently high reaction speed, and is preferably not exceeding 200° C, particularly not exceeding 180° C in order to substantially avoid coloration of aliphatic polyester by oxidation or decomposition of the generated aliphatic polyester. Also the reaction time can be arbitrarily selected within a range not affecting the quality of the generated aliphatic polyester.

The ring-opening polymerization of ε-caprolactone can also be executed in a solvent. The solvent is preferably an inactive solvent not reacting with ε-caprolactone, polymerization catalyst or polymerization initiator, selected from aromatic hydrocarbons such as toluene or xylene, or aliphatic or alicyclic hydrocarbons such as hexane or cyclohexane. Preferably such solvent is substantially anhydrous. The reaction temperature is within a range from 0° C to the boiling point of the solvent, preferably within a range of 0° C to 100° C.

The weight-average molecular weight of aliphatic polyester obtained by the ring-opening polymerization of ε-caprolactone is preferably 1,000 or higher, particularly 30,000 or higher in terms of polystyrene and preferably 1,000,000 or lower, particularly 500,000 or lower in terms of polystyrene.

The aliphatic polyester of the present invention thus obtained can be utilized in various industrial fields by modifying the weight-average molecular weight or the functional radical contained therein. For example, the aliphatic polyester of a weight-average molecular weight of 1,000 to 5,000 utilizing glycol as a polymerization initiator is extremely useful, exploiting the presence of a hydroxyl radical therein, as a raw material for polyurethane or paints. Also the aliphatic polyester having a weight-average molecular weight exceeding 50,000 has a practical mechanical strength and is usable in plastic molded articles, films or hot-melt adhesives. The molding can be executed, for example, by compression molding, injection molding, extrusion molding, mold casting or transfer molding utilizing a mold.

Also the aliphatic polyester of the present invention may be mixed, within a range not affecting the object of the present invention, with another resinous component, a rubber component, a heat resistance stabilizer, a flame retarding agent, a slipping agent, an antiblocking agent, an anticlouding agent, a friction reducing agent, a filler, a dye, a pigment, natural oil, synthetic oil or wax. The mixing ratio is not particularly limited and can be suitably determined.

In the following, the present invention will be further clarified by way of examples, but the present invention is by no means limited to such examples.

### (Example 1)

500 parts by weight of starch (supplied by Wako Pure Chemical Industries Co.) were put into 4,500 parts by weight of water and were dissolved under heating. Then 5,000 parts by weight of 3 mol/l sulfuric acid were added and reacted under agitation for 5 hours at 80°C. After the reaction, the aqueous solution was neutralized by the addition of anhydrous sodium carbonate, then was passed through a column of the ion exchange resin (Amberlite IR-120B, supplied by Organo Co.) and the solvent was distilled off. Then the reaction mixture was separated and purified to obtain 300 parts by weight of glucose.

The ¹³C-NMR (100 MHz, internal standard DMSO-d₆) of the synthesized glucose was measured with FT-NMR DPX400 (manufactured by Bruker Inc.) to obtain chemical shfts δ (ppm) as follows:
α-type: 92.12, 73.04, 72.29, 71.80, 70.58, 61.20
β-type: 96.79, 76.70, 76.59, 74.78, 70.30, 61.00

8,000 parts by weight of 12% aqueous solution of barium carbonate were saturated with carbon dioxide, and 330 parts by weight of bromine and 300 parts by weight of the glucose were added and agitated for 30 minutes at 25°C to obtain 250 parts by weight of gluconolactone represented by the following chemical formula (III):

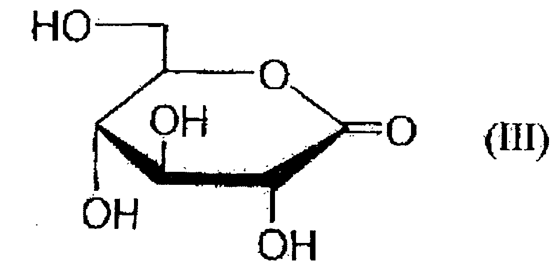

The ¹³C-NMR (100 MHz, internal standard DMSO-d₆) of the synthesized gluconolactone was measured to obtain chemical shfts δ (ppm) as follows:
gluconolactone: ¹³C-NMR (100 MHz, internal standard DMSO-d₆) δ (ppm): 171.88, 81.23, 73.79, 71.43, 67.82, 60.14

87 parts by weight of red phosphorus and 250 parts by weight of the gluconolactone were added to 12,000 parts by weight of hydroiodic acid (55 mass%), and were refluxed for 20 hours at 130°C. The reaction mixture was filtered, then the filtrate was extracted with ether and the extract was washed with 5% aqueous solution of sodium hydrosulfite. After the solvent ether was distilled off, distillation under a reduced pressure was executed to obtain 147 parts by weight of caproic acid.

The ¹³C-NMR (100 MHz, internal standard CDCl₃) of the synthesized caproic acid was measured to obtain chemical shfts δ (ppm) as follows:
caproic acid: ¹³C-NMR (100 MHz, CDCl₃) δ (ppm): 180.78, 34.24, 31.36, 24.49, 22.42, 13.90

147 parts by weight of the caproic acid were added to 1,000 parts by weight of 90% sulfuric acid saturated with chlorine and were reacted for 6 hours at 25°C to obtain 95 parts by weight of 6-chlorocaproic acid.

The ¹³C-NMR (100 MHz, internal standard CDCl₃) of the synthesized 6-chlorocaproic acid was measured to obtain chemical shfts δ (ppm) as follows:
6-chlorocaproic acid: ¹³C-NMR (100 MHz, CDCl₃) δ (ppm): 180.18, 44.69, 33.93, 32.25, 26.36, 23.98

95 parts by weight of the 6-chlorocaproic acid were boiled with an aqueous solution of the equivalent amount of sodium hydroxide to obtain 69 parts by weight of ε-caprolactone.

The ¹³C-NMR (100 MHz, internal standard CDCl₃) of the synthesized ε-caprolactone was measured to obtain chemical shfts δ (ppm) as follows:
ε-caprolactone: ¹³C-NMR (100 MHz, CDCl₃) δ (ppm): 176.23, 69.30, 34.56, 29.35, 28.93, 22.98

69 parts by weight of the ε-caprolactone were heated to 155°C in a nitrogen atmosphere, and 0.21 parts by weight of tri-iso-propoxy-aluminum as a polymerization catalyst and 0.41 parts by weight of diethylene glycol as a polymerization initiator were added to execute ring-opening polymerization thereby obtaining aliphatic polyester. The polymerization time was 10 hours. The obtained aliphatic polyester shows a weight-average molecular weight of 300,000 in terms of polystyrene and an average degree of polymerization of 2,630.

The ¹H-NMR (400 MHz, internal standard CDCl₃) and ¹³C-NMR (100 MHz, internal standard CDCl₃) of the synthesized aliphatic polyester were measured to obtain chemical shfts δ (ppm) as follows:
aliphatic polyester: ¹H-NMR (400 MHz, CDCl₃) δ (ppm): 1.36 to 1.42 (2H, m), 1.61 to 1.69 (4H, m), 2.31 (2H, t), 4.06 (2H, t)
aliphatic polyester: ¹³C-NMR (100 MHz, CDCl₃) δ (ppm): 24.59, 25.54, 28.36, 34.12, 64.16, 173.56

These results of measurement confirmed that the desired aliphatic polyester was synthesized.

### (Example 2)

300 parts by weight of glucose, obtained in the same manner as in the example 1, were oxidized and hydrolysed in 2,500 parts by weight of 27N sulfuric acid saturated with bromine to obtain 290 parts by weight of gluconic acid represented by the following chemical formula (IV): 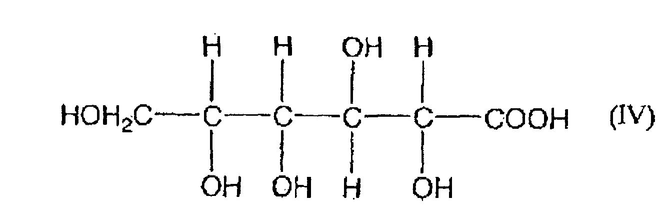 100 parts by weight of red phosphorus and 290 parts by weight of the gluconic acid were added to 14,000 parts by weight of hydroiodic acid (55 mass%) and were refluxed for 20 hours at 130°C. Then the subsequent process was conducted in the same manner as in the example 1 to obtain 155 parts by weight of caproic acid.

155 parts by weight of the caproic acid were added to 1,000 parts by weight of 90% sulfuric acid saturated with chlorine and were reacted for 6 hours at 25°C to obtain 100 parts by weight of 6-chlorocaproic acid.

100 parts by weight of the 6-chlorocaproic acid were boiled with an aqueous solution of the equivalent amount of sodium hydroxide to obtain 73 parts by weight of ε-caprolactone.

73 parts by weight of ε-caprolactone were heated to 160°C in a nitrogen atmosphere, and 0.22 parts by weight of di-iso-propyl zinc as a polymerization catalyst and 0.44 parts by weight of 1,4-butanediol as a polymerization initiator were added to execute ring-opening polymerization thereby obtaining aliphatic polyester. The polymerization time was 10 hours. The obtained aliphatic polyester showed a weight-average molecular weight of 250,000 in terms of polystyrene and an average degree of polymerization of 2,190.

The measurement of ¹H-NMR and ¹³C-NMR provided spectra similar to those in the example 1, confirming that the desired aliphatic polyester was synthesized.

### (Evaluation of physical properties)

The aliphatic polyesters synthesized in the examples 1 and 2 were subjected to evaluation of various physical properties, of which results are shown in Table 1. Also as a reference example 1, Celgreen (polycaprolactone plastic P-H7 manufactured by Daicel Chemical Industries, Co.) was included in the comparative evaluation.

**Table 1**

| | Example 1 | Example 2 | Reference Example 1 |
|---|---|---|---|
| Tensile yield strength (JIS)K7113 Pa | 0.25 | 0.22 | 0.20 |
| Tensile modulus (JIS)K7113 Pa | 2.45 | 2.30 | 2.25 |
| Bending strength (JIS)K7203 Pa | 0.43 | 0.40 | 0.37 |
| Bending modulus (JIS)K7203 Pa | 5.00 | 4.75 | 4.41 |

These results indicate that the aliphatic polyesters synthesized in the examples 1 and 2 have physical properties equivalent or superior to those of the aliphatic polyester P-H7 of Daicel Chemical of the reference example 1 excellent in the strength and elongation, and can be. satisfactorily used as a substitute for the conventionally known plastics derived from petroleum.

As explained in the foregoing, the present invention enables to produce aliphatic polyester by the ring-opening polymerization of ε-caprolactone obtained from starch via glucose, and such aliphatic polyester has sufficient physical properties such as mechanical strength and can be utilized as a substitute for plastic molded products. Furthermore, this fact opens up a way of obtaining high-quality plastic materials from starch instead of petroleum, thereby establishing starch as an efficient resource.

## Claims

1. A method of producing an aliphatic' polyester represented by the following formula (I): 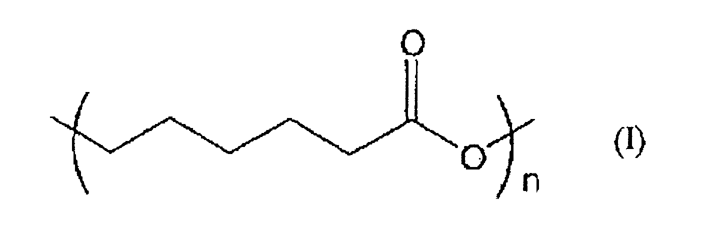 wherein n stands for an integer with a range of 5 to 10,000, the method comprising the steps of:
(i) hydrolyzing starch to obtain glucose;
(ii) oxidizing said glucose to obtain gluconolactone;
(iii) reducing said gluconolactone to obtain caproic acid;
(iv) chlorinating said caproic acid to obtain 6-chlorocaproic acid;
(v) cyclizing said 6-chlorocaproic acid to obtain ε-caprolactone represented by the following formula (II): 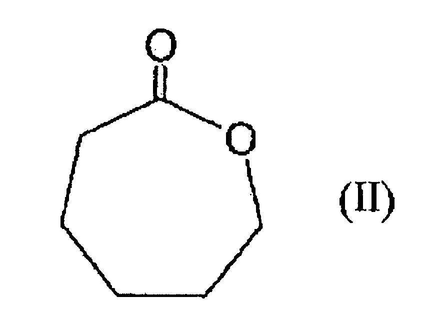 and
(vi) executing ring-opening polymerization of said ε-caprolactone.

2. A method of producing an aliphatic polyester represented by the following formula (I): 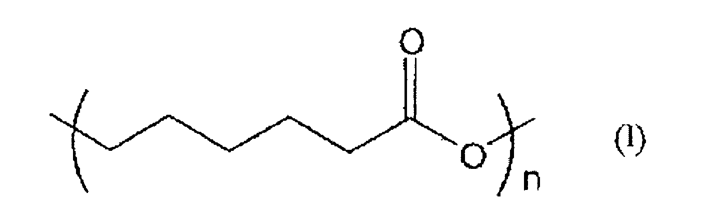 wherein n stands for an integer with a range of 5 to 10,000, the method comprising the steps of:
(i) hydrolyzing starch to obtain glucose;
(ii) oxidizing said glucose to obtain gluconic acid;
(iii) reducing said gluconic acid to obtain caproic acid;
(iv) chlorinating said caproic acid to obtain 6-chlorocaproic acid;
(v) cyclizing said 6-chlorocaproic acid to obtain ε-caprolactone represented by the following formula (II): 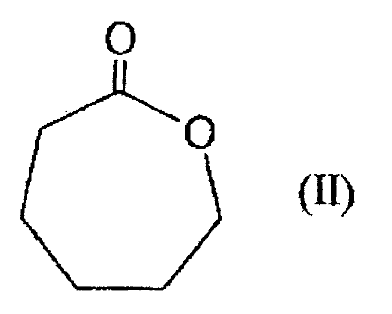 and
(vi) executing ring-opening polymerization of said ε-caprolactone.

3. The method according to claim 1 or 2, wherein the step of obtaining glucose from starch is executed by hydrolysis utilizing an acid.

4. The method according to claim 1, wherein the step of obtaining gluconolactone from glucose is executed by bromine oxidation.

5. The method according to claim 2, wherein the step of obtaining gluconic acid from glucose is executed by oxidation utilizing bromine and concentrated sulfuric acid.

6. The method according to claim 1, wherein the step of obtaining caproic acid from gluconolactone is executed by a reducing reaction utilizing hydroiodic acid and red phosphorus.

7. The method according to claim 2, wherein the step of obtaining caproic acid from gluconic acid is executed by a reducing reaction utilizing hydroiodic acid and red phosphorus.

8. The method according to claim 1 or 2, wherein the step of obtaining 6-chlorocaproic acid from caproic acid is executed by a chlorination reaction utilizing chlorine and concentrated sulfuric acid.

9. The method according to claim 1 or 2, wherein the step of obtaining ε-caprolactone from 6-chlorocaproic acid is executed by a cyclization reaction utilizing an aqueous solution of sodium hydroxide.

10. The method according to claim 1 or 2, wherein the step of ring-opening polymerization of ε-caprolactone is executed by a ring-opening polymerization utilizing a polymerization catalyst and a polymerization initiator.
